# EUROPEAN PATENT APPLICATION

(11) **EP 1 891 971 A1**
(43) Date of publication of application: **27.02.2008**
(21) Application number: 06712691.2
(22) Date of filing: 31.01.2006
(51) Int. Cl.: A61K 45/00, A61K 31/4045, A61P 3/10, A61P 9/10, A61P 43/00, C07D 209/44

(54) **MEDICINAL COMPOSITION FOR AMELIORATING OR TREATING GLUCOSE INTOLERANCE, BORDERLINE DIABETES, INSULIN RESISTANCE AND HYPERINSULINEMIA CONTAINING HYPOGLYCEMIC AGENT**

(30) Priority: 31.01.2005 JP 2005023289
(71) Applicant: Ajinomoto Co., Inc., Tokyo 104-8315 (JP)
(72) Inventor: KITAHARA, Yoshiro, -ku, Kawasaki-shi, Kanagawa, 2108681 (JP); MIURA, Kyoko, -ku, Kawasaki-shi, Kanagawa, 2108681 (JP)
(74) Representative: Nicholls, Kathryn Margaret
(86) International application number: PCT/JP2006/301548
(87) International publication number: WO 2006/080524

(57) **Abstract**

The present invention provides a pharmaceutical composition containing a hypoglycemic agent(s) as an active ingredient for improving or treating impaired glucose tolerance, borderline diabetes, insulin resistance or hyperinsulinemia. The present invention further provides a pharmaceutical composition for preventing or delaying the progression from impaired glucose tolerance, borderline diabetes, insulin resistance or hyperinsulinemia to diabetes mellitus or metabolic syndromes; or the progression to the state easy to develop macrovascular disorders, cardiovascular events or ischemic heart disease.

## Description

### Technical Field of the Invention

The present invention relates to therapeutic agents for treating borderline diabetes. It also relates to therapeutic agents for preventing development of diabetes or macrovascular disorders such as atherosclerosis and myocardial infarction and progression from borderline diabetes to such diseases.

### Background of the Invention

According to the survey on the actual conditions regarding diabetes conducted in November 2002 by the Ministry of Health, Labour and Welfare, it is presumed that the individuals strongly suspected of having diabetes in Japan including patients under treatment are about 7.4 million. It is also reported that, in addition to the above individuals, the individuals regarding whom it cannot be denied that there is the possibility of having diabetes are about 8.8 million, and the total number of the individuals strongly suspected of having diabetes and the individuals regarding whom it cannot be denied that there is the possibility of having diabetes is about 16.2 million (Non-patent Literature 1). The individuals regarding whom it cannot be denied that there is the possibility of having diabetes mean those whose hemoglobin A1c is 5.6% or higher and less than 6.1% and that are not under diabetic treatments.

Recent years, it is being known that diabetic patients have a significantly higher risk of developing cardiovascular events caused by atherosclerosis as compared with nondiabetic patients. Therefore, the final object of the recent diabetic treatments is not only to decrease the blood glucose but also to focus on inhibiting the cardiovascular events caused by atherosclerosis, which follow diabetes. American Diabetes Association (ADA), World Health Organization (WHO) and Japan Diabetes Society (JDS) lately announced new diagnostic criteria for diabetes, taking into consideration the achievements of clinical and epidemiologic studies. According to them, as for the blood glucose level, one is diagnosed as diabetes when either one of the values that: fasting blood glucose ≧ 126mg/dl; casual blood glucose ≧ 200mg/dl; or blood glucose two hours after the 75g oral glucose tolerance test (OGTT) ≧ 200mg/dl is seen (Diabetes Care 20: 1183 (1997), Diabet Med 15: 539 (1998), and Diabetes 42: 385 (1999); Non-patent Literatures 2 to 4). However, since the casual blood glucose dynamically varies due to the timing of eating meals and 75g OGTT is time-consuming, the measurement of the fasting blood glucose has been given priority from the viewpoint of ease and economic efficiency. Under these situations, the individuals regarding whom it cannot be denied that there is the possibility of having diabetes or those with borderline diabetes have a feature that, although their fasting blood glucose is within a normal range, their blood glucose level after eating is high. Therefore, it is often the case that they are not diagnosed as diabetes and not under treatment. In the prospective study in Europe wherein about 25,000 cases were the subject and followed up for about 7 years in average (the DECODE study), it was indicated that the increase of the blood glucose two hours after eating strongly relates to the increase of the risk of death (Lancet 354: 617 (1999), Non-patent Literature 5). Further, also in the epidemiologic research conducted in Funagata-machi, Yamagata-ken, Japan, it has been reported that hyperglycemia after eating has the risk of developing myocardial or brain infarction twice as high as a healthy individual (Diabetes Care 22: 920 (1999), Non-patent Literature 6). The more important thing is that, in these researches, it was indicated that the individual with so-called borderline diabetes, that is, whose fasting blood glucose is in the normal level and blood glucose after eating is 140mg/dl or higher and less than 200mg, has the increased risk of developing cardiovascular events. Thus, it has been reported that treatment at an earlier stage before the diagnosis of diabetes is important.

The individuals regarding whom it cannot be denied that there is the possibility of having diabetes or those with borderline diabetes also have a feature that the insulin level in the plasma is high in addition to the high blood glucose level after eating. This hyperinsulinemia and insulin resistance caused thereby are known as risk factors progressing metabolic syndromes including atherosclerosis. As the factors of inducing hyperinsulinemia, it is believed that they are mainly the reduction of the prompt secretion of insulin after eating as seen in a healthy individual and the so-called delayed excess secretion thereof. Thus, recovery of the prompt secretion of insulin after eating is the important treatment that should be conducted from the stage at which individuals have borderline diabetes.

Meanwhile, oral hypoglycemic agents of glinides comprising mitiglinide, nateglinide and repaglinide are widely used as fast-acting insulin secreragogues. These agents inhibit the increase of the blood glucose after eating by improving the prompt secretion of insulin after eating that is reduced in patients with diabetes. However, it has not yet been clinically checked whether the administration of these agents to those with borderline diabetes is effective against the progression of the subsequent pathology. The various research results are clarifying that about 8.8 million of the individuals regarding whom it cannot be denied that there is the possibility of having diabetes have a higher risk of developing atherosclerosis although they are not under diabetic treatments. Therefore, it is a very important point to provide these individuals with the treatment of administering therapeutic agents at an early stage in order to inhibit progressing metabolic syndromes by improving hyperinsulinemia and insulin resistance.
Non-patent Literature 1: Report of the survey on the actual conditions regarding diabetes 2002, the Office of Lifestyle-related diseases, the General Affairs Division of the Health Service Bureau, the Ministry of Health, Labour and Welfare, 2003
Non-patent Literature 2: Diabetes Care 20: 1183 (1997)
Non-patent Literature 3: Diabet Med 15: 539 (1998)
Non-patent Literature 4: Diabetes 42: 385 (1999)
Non-patent Literature 5: Lancet 354: 617 (1999)
Non-patent Literature 6: Diabetes Care 22: 920 (1999)

### Disclosure of the Invention

The object of the present invention is to provide therapeutic agents for inhibiting the progression to metabolic syndromes including atherosclerosis by improving hyperinsulinemia, insulin resistance or hyperglycemia after eating, and said improvements achieved by recovering the prompt secretion of insulin after eating of the individuals with IGT or borderline diabetes.

The inventors thoroughly studied the above problem and found that the administration of glinide type agents including mitiglinide or the like from the stage of IGT improves hyperinsulinemia and insulin resistance, and the present invention has been completed based on this finding.

Namely, the inventors focused on the recovering effect of glinide type agents on the prompt secretion of insulin after eating, and found that the administration of the agents from the stage of IGT improves the delayed excess insulin secretion after eating to improve hyperinsulinemia and also improves the general insulin sensitivity to be able to decrease the blood glucose in less insulin content.

Specifically, the inventors administered mitiglinide as a glinide type agent to Zucker Fatty rats that are the model animal of IGT, and investigated their general insulin sensitivity and the circadian changes of their blood glucose and insulin.

As a result, they found that the administration of mitiglinide for four weeks inhibits the excess insulin secretion after eating and improves the general insulin sensitivity.

Based on the above finding, the inventors completed the present invention that relates to agents for preventing or treating the progression of borderline diabetes.

Namely, the present invention provides a pharmaceutical composition for improving or treating impaired glucose tolerance, borderline diabetes, insulin resistance or hyperinsulinemia, containing a hypoglycemic agent(s) as an active ingredient.

The present invention also provides a pharmaceutical composition for preventing or delaying the progression from impaired glucose tolerance, borderline diabetes, insulin resistance or hyperinsulinemia to diabetes mellitus (DM) or metabolic syndromes, containing a hypoglycemic agent(s) as an active ingredient.

The present invention further provides a pharmaceutical composition for preventing or delaying the progression from impaired glucose tolerance, borderline diabetes, insulin resistance or hyperinsulinemia to the state easy to develop macrovascular disorders, cardiovascular events or ischemic heart disease, containing a hypoglycemic agent(s) as an active ingredient.

The present invention additionally provides use of a hypoglycemic agent(s) for the preparation of a pharmaceutical composition for improving or treating impaired glucose tolerance, borderline diabetes, insulin resistance or hyperinsulinemia.

The present invention further additionally provides use of a hypoglycemic agent(s) for the preparation of a pharmaceutical composition for preventing or delaying the progression from impaired glucose tolerance, borderline diabetes, insulin resistance or hyperinsulinemia to diabetes mellitus or metabolic syndromes.

The present invention further additionally provides use of a hypoglycemic agent(s) for the preparation of a pharmaceutical composition for preventing or delaying the progression from impaired glucose tolerance, borderline diabetes, insulin resistance or hyperinsulinemia to the state easy to develop macrovascular disorders, cardiovascular events or ischemic heart disease.

Due to the present invention, it is possible to provide the therapeutic agents for preventing or treating the progression of the borderline diabetic pathology to patients with borderline diabetes, who are not under treatment at present though they have a higher risk of developing vascular complications such as arterial sclerosis.

### Brief Description of the Drawings

Figure 1 shows the result of investigating the effect of the treatment by mitiglinide on the circadian changes of the blood glucose.
Figure 2 shows the result of investigating the effect of the treatment by mitiglinide on the circadian changes of insulin.
Figure 3 shows the result of the insulin tolerance test.

### Best Mode for Carrying out the Invention

It is preferable that hypoglycemic agents decrease the insulin level in the blood plasma one hour after the administration thereof by 15ng/mL or more as compared with the case where the pharmaceutical composition of the present invention is not administered. It is also preferable that hypoglycemic agents decrease the insulin level in the blood plasma one hour after the administration thereof to 45ng/mL or less.

Specifically, it is possible to use α-glucosidase inhibitors (α-GI), fast-acting insulin secretagogues (glinide type agents), sulfonylurea agents (SU agents), biguanides (BG) and the like.

Examples of α-glucosidase inhibitors include voglibose and acarbose.

Examples of fast-acting insulin secretagogues include (2S)-2-benzyl-4-[(3aR,7aS)-octahydro-2H-isoindole-2-yl]-4-oxobutanoic acid (general name: mitiglinide), N-(trans-4-isopropylcyclohexylcarbonyl)-D-phenylalanine (general name: nateglinide), (S)-2-ethoxy-4-{2-[[3-methyl-1-[2-(1-piperidinyl)phenyl]butyl]amino]-2-oxoethyl}benzoic acid (general name: repaglinide) and the like.

Examples of sulfonylurea agents (SU agents) include tolbutamide, chlorpropamide, tolazamide, acetohexamide, gliclazide, 4-chloro-N-[(1-pyrrohdinylamino)carbonyl]-benzenesulfone amide (glyco-pyramide), 1-butyl-3-metanilylurea, glipizide, gliquidone and the like.

Examples of thiazolidine agents include glitazone agents such as pioglitazone and rosiglitazone.

Examples of biguanides include metformin, buformin and the like.

It is possible to use crystalline forms, solvates, and pharmaceutically acceptable salts of the above hypoglycemic agents. Among them, the fast-acting insulin secretagogues are preferable, and mitiglinide or pharmaceutically acceptable salts thereof are particularly preferable.

The composition of the present invention is effective in: improving or treating impaired glucose tolerance (IGT), borderline diabetes, insulin resistance or hyperinsulinemia; preventing or delaying the progression to diabetes mellitus or metabolic syndromes from the above states or diseases; and particularly preventing, delaying or treating the progression to the state easy to develop cardiovascular events, ischemic heart disease and macrovascular disorders such as angina, myocardial infarction and arterial sclerosis, induced by the above states or diseases, and especially by insulin resistance or hyperinsulinemia. Further, the composition of the present invention is particularly effective in: improving or treating IGT, insulin resistance or hyperinsulinemia; and preventing or delaying the progression to diabetes or metabolic syndromes from IGT. Among them, it is especially effective in improving or treating IGT or hyperinsulinemia.

Meanwhile, in the present specification, the term "metabolic syndrome(s)" indicates the meaning as known in the art. Specifically, it indicates the state wherein three or more risk factors (insulin resistance, obesity, high triglyceride level, low HDL cholesterol level, and high-blood pressure) of cardiovascular diseases based on insulin resistance accumulate, which is the definition proposed by Adult Treatment Panel III of National Cholesterol Education Program, 2001.

The composition of the present invention preferably contains a hypoglycemic agent(s) of which single administered dose is less than 20mg and more preferably 10mg or less.

The composition of the present invention is administered orally or parenterally, e.g., intramuscularly, subcutaneously or intravenously. The oral administration is preferable. The administered dose for the above purposes is determined based on the desired therapeutic effects, administration method, treatment period, age and body weight of the patient. In the oral or parenteral route, it is preferable to use 30 to 60mg, more preferably 15 to 30mg and further more preferably 7.5 to 15mg in the case of the oral administration, as the usual administered dose to an adult per a day; and 100µg to 60mg or less in the case of the parenteral administration. It is preferable to administer the composition just before each meal.

Further, when preparing the composition of the present invention as oral preparations, excipients and, if necessary, binders, disintegrating agents, lubricants, coloring agents and flavoring agents are added thereto. Then, the mixture is formulated into tablets, dispersants, pills, granules, capsules, suppositories, solutions, sugar-coated agents, depot agents, or syrups in accordance with the ordinary method. Examples of excipients include lactose, corn starch, sucrose, glucose, sorbit, and crystalline cellulose. Examples of binders include polyvinyl alcohol, polyvinyl ether, ethylcellulose, methylcellulose, gum Arabic, tragacanth, gelatin, shellac, hydroxypropyl cellulose, hydroxypropyl starch, and polyvinyl pyrrolidone. Examples of disintegrating agents include starch, agar, gelatin powder, crystalline cellulose, calcium carbonate, sodium hydrogen carbonate, calcium citrate, dextran, and pectin. Examples of lubricants include magnesium stearate, talc, polyethylene glycol, silica, and hardened vegetable oil. Examples of coloring agents include those of which addition to pharmaceutical compositions are accepted. Examples of flavoring agents include cocoa powder, menthol, aromatic acids, mint oil, borneol, and cinnamon powder. It is of course sufficient to arbitrarily coat these tablets or granules with sugar, gelatin or the like, if necessary.

When preparing injectable solutions, pH adjusters, buffers, stabilizing agents, and preservatives can be added if necessary, and subcutaneous, intramuscular, or intravenous injectable solutions can be prepared in accordance with the ordinary method.

### Examples

Next, Examples will further illustrate the present invention. They only explain the present invention and do not particularly limit the invention.

### Example 1

### <Effects on the pathologic progression of Zucker Fatty rats>

The effectiveness of mitiglinide was examined by using Zucker Fatty (ZF) rats, which have the feature of obesity and insulin resistance. It is known that though ZF rats have hyperinsulinemia and impaired glucose tolerance, their fasting blood glucose is within a normal range and, therefore, their pathology is similar to IGT in human being (Br J Pharmacol 125, 1708-14, 1998).

Male Zucker Fatty rats of 6 weeks old were introduced and acclimatized to the restricted feeding of one hour each (9:00 to 10:00 and 15:00 to 16:00) twice per a day. After acclimatization for one week, the rats were randomly divided into two groups, and drugs were administered to them for four weeks. As the drugs, mitiglinide suspended in 0.5% methylcellulose was forcibly orally administered to one group to become a dose of 3mg/kg, and only 0.5% methylcellulose solution that is a dose vehicle was forcibly orally administered to the other group just before each meal twice per a day. The circadian changes of their blood glucose and insulin that occurred under the restricted feeding were measured upon starting the administration and in the fourth week after its start. The drugs were not administered on the measurement day, and their blood was collected through the caudal vein before morning feeding (9:00), one hour after the feeding (10:00), two hours after the feeding (11:00), before afternoon feeding (15:00), one hour after the feeding (16:00), and two hours after the feeding (17:00). Then, their blood glucose and insulin were measured in accordance with the routine method.

On another day of the fourth week, the insulin tolerance test (ITT) was conducted in order to examine the degree of improvement of the general insulin resistance. Commercially available insulin preparation (NOVORIN R, NOVO) diluted to 0.5U/mL with a normal saline solution was subcutaneously injected to the back of ZF rats fasted overnight to become 0.5U/kg. Their blood was collected through the caudal vein 15, 30, 60, 120 and 180 minutes after the administration to measure their blood glucose. The blood glucose was measured in accordance with the routine method.

### (Results)

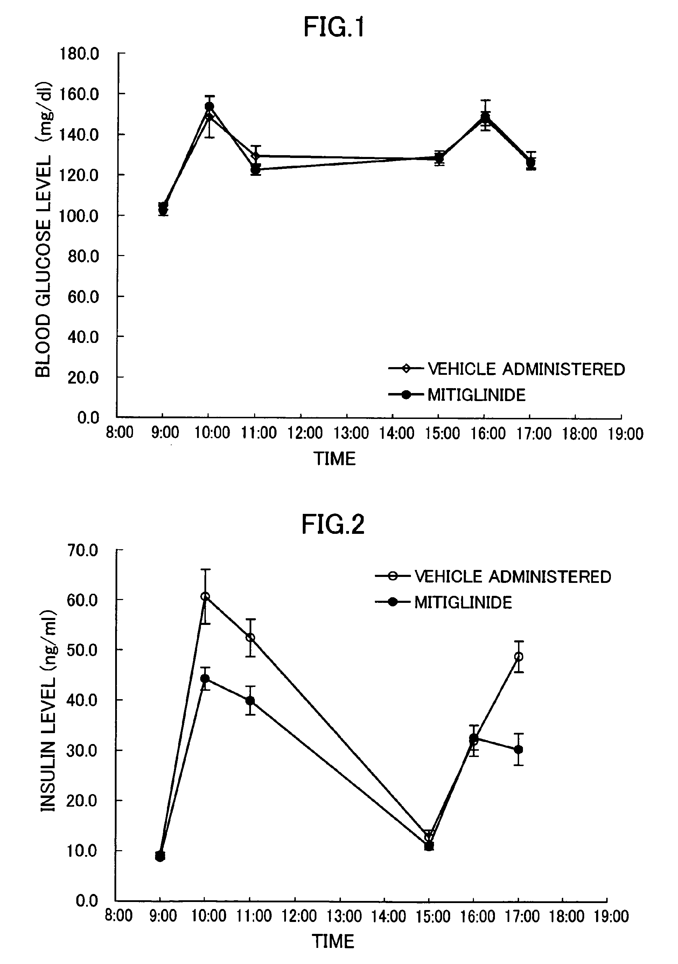

The results of the effects of the treatment by mitiglinide on the circadian changes of the blood glucose and insulin were shown in Figures 1, 2 and 3, and Tables 1 and 2. Figure 1 shows the circadian changes of the blood glucose when feeding 9:00 to 10:00 and 15:00 to 16:00. There was no change in the circadian changes of the blood glucose in both groups. Similarly, the sums of the blood glucose levels in a day were almost the same as shown in Table 1. On the other hand, as shown in Figure 2, which shows the insulin level in the blood plasma at that time, the decrease of the insulin level was clearly seen in the mitiglinide administered group. In the sums of the insulin levels in a day as shown in Table 1, the insulin level of the mitiglinide administered group was significantly lower than that of the other. These results indicate that, due to the improvement of insulin resistance by the long-period treatment with mitiglinide, the increase of the blood glucose can be inhibited in less insulin content.

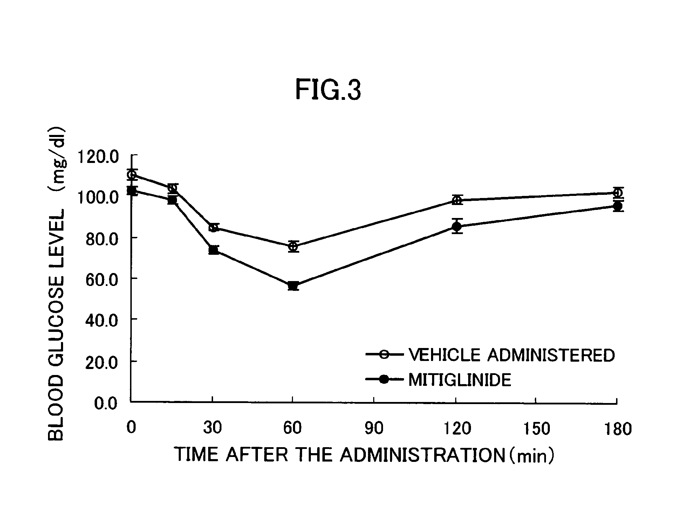

In response to the above results, ITT was conducted in order to more precisely quantify the degree of improvement of the general insulin sensitivity. The blood glucose transiently lowers by injecting insulin, but it does not notably lower by injecting insulin in case of having strong insulin resistance. As shown in Figure 3, the blood glucose of ZF rats only lowered by about 70% of the level before the administration in 60 minutes after the administration of insulin. However, the blood glucose lowered by about 50% of the level before the administration in 60 minutes after the administration of insulin due to the treatment by mitiglinide. In comparing the sums of the blood glucose levels after administering insulin, the level was clearly low in the mitiglinide administered group as shown in Table 2 and, therefore, the improvement of insulin resistance was indicated.

From the above experimental results, it was clarified that the treatment by mitiglinide from the stage of IGT improves hyperinsulinemia or insulin resistance that is thought to be a factor progressing arterial sclerosis. Thus, it was thought that said treatment can inhibit the progression from IGT to DM or so-called metabolic syndromes.

**Table 1**

| | Sum of the circadian changes of blood glucose | Sum of the circadian changes of insulin |
|---|---|---|
| Dose vehicle group | 1061±37mg · hr/dl | 285±20ng · hr/ml |
| Mitiglinide group | 1050±20mg · hr/dl | 224±11ng · hr/ml |

**Table 2**

| | Sum of blood glucose |
|---|---|
| Dose vehicle group | 279±6mg · hr/dl |
| Mitiglinide group | 243±7mg · hr/dl |

## Claims

1. A pharmaceutical composition for improving or treating impaired glucose tolerance, borderline diabetes, insulin resistance or hyperinsulinemia, containing a hypoglycemic agent(s) as an active ingredient.

2. A pharmaceutical composition for preventing or delaying the progression from impaired glucose tolerance, borderline diabetes, insulin resistance or hyperinsulinemia to diabetes mellitus or metabolic syndromes, containing a hypoglycemic agent(s) as an active ingredient.

3. A pharmaceutical composition for preventing or delaying the progression from impaired glucose tolerance, borderline diabetes, insulin resistance or hyperinsulinemia to the state easy to develop macrovascular disorders, cardiovascular events or ischemic heart disease, containing a hypoglycemic agent(s) as an active ingredient.

4. The pharmaceutical composition according to any one of claims 1 to 3, wherein the hypoglycemic agent(s) decreases the insulin level in the blood plasma one hour after the administration thereof by 15ng/mL or more as compared with the case where the pharmaceutical composition of the present invention is not administered.

5. The pharmaceutical composition according to any one of claims 1 to 3, wherein the hypoglycemic agent(s) decreases the insulin level in the blood plasma one hour after the administration thereof to 45ng/mL or less.

6. The pharmaceutical composition according to any one of claims 1 to 5, wherein the hypoglycemic agent(s) is selected from the group consisting of α -glucosidase inhibitors, fast-acting insulin secretagogues, sulfonylurea agents, thiazolidine agents and biguanides.

7. The pharmaceutical composition according to claim 6, wherein the hypoglycemic agent(s) is the fast-acting insulin secretagogue(s).

8. The pharmaceutical composition according to claim 7, wherein the fast-acting insulin secretagogue(s) is (2S)-2-benzyl-4-[(3aR,7aS) -octahydro-2H-isoindole-2-yl]-4-oxobutanoic acid, N-(trans-4-isopropylcyclohexylcarbonyl)-D-phenylalanine, (S)-2-ethoxy-4-{2-[[3-methyl-1-[2-(1-piperidinyl)phenyl]butyl]amino]-2-oxoethyl} benzoic acid or pharmaceutically acceptable salts thereof.

9. The pharmaceutical composition according to claim 8, wherein the fast-acting insulin secretagogue(s) is (2S)-2-benzyl-4-[(3aR,7aS) -octahydro-2H-isoindole-2-yl]-4-oxobutanoic acid or pharmaceutically acceptable salts thereof.

10. Use of a hypoglycemic agent(s) for the preparation of a pharmaceutical composition for improving or treating impaired glucose tolerance, borderline diabetes, insulin resistance or hyperinsulinemia.

11. Use of a hypoglycemic agent(s) for the preparation of a pharmaceutical composition for preventing or delaying the progression from impaired glucose tolerance, borderline diabetes, insulin resistance or hyperinsulinemia to diabetes mellitus or metabolic syndromes.

12. Use of a hypoglycemic agent(s) for the preparation of a pharmaceutical composition for preventing or delaying the progression from impaired glucose tolerance, borderline diabetes, insulin resistance or hyperinsulinemia to the state easy to develop macrovascular disorders, cardiovascular events or ischemic heart disease.
